# EUROPEAN PATENT APPLICATION

(11) **EP 1 795 130 A1**
(43) Date of publication of application: **13.06.2007**
(21) Application number: 06025087.5
(22) Date of filing: 05.12.2006
(51) Int. Cl.: A61B 8/08, A61B 19/00

(54) **Ultrasound system for interventional treatment**

(30) Priority: 05.12.2005 KR 20050117502
(71) Applicant: MEDISON CO., LTD., Kangwon-do 250-870 (KR)
(72) Inventor: Kim, Ceol An, Gangnam-gu, Seoul 135-280 (KR); Song, Young Seuk, Gangnam-gu, Seoul 135-280 (KR)
(74) Representative: Lorenz, Markus

(57) **Abstract**

An ultrasound system includes an ultrasound diagnostic unit for transmitting ultrasound signals to a target object and producing ultrasound image signals; a wide-area image signal providing unit for providing wide-area image signals for a relatively wide area; an image processor for forming a reconstruction image by combining an ultrasound image and a wide-area image based on the ultrasound image signals and the wide-area image signals; a user interface for selecting at least one lesion in the target object and at least one medical needle insertion point by a user; a path setting unit for setting at least one medical needle insertion path based on the lesion and the medical needle insertion point; and a display unit for displaying the reconstruction image showing positions of the lesion and the medical needle insertion point and the medical needle insertion path, the display unit further being configured to display the ultrasound image.

## Description

The present application claims priority from Korean Patent Application 10-2005-117502, filed on December 5, 2005, the entire subject matter of which is incorporated herein by reference.

### BACKGROUND

### 1. Field

The present invention generally relates to an ultrasound system, and more particularly to an ultrasound system for interventional treatment.

### 2. Background

Improvement of medical techniques makes it possible to perform treatment or examination by inserting a medical needle such as ablator needle or biopsy needle into a lesion region via a minimum incision without an entire incision while referring to an image. Such treatment, which is performed while observing internal structures of a body by using a diagnostic imaging system, is referred to as "image-based treatment" or "interventional treatment". Specifically, the interventional treatment is performed by directing the medical needle to the lesion to be treated or examined through a skin while referring to images during the treatment, the images obtained by employing a computerized tomography (CT) scanner generally used in a radiology department or a magnetic resonance imaging (MRI) system. Compared to surgical treatment requiring incision, the interventional treatment has advantages of cost reduction and effectiveness in that general anesthesia is unnecessary and patients have less pain and recover speedily.

However, it is difficult to obtain real-time images in the CT scanner or the MRI system. Especially, when the interventional treatment is performed by using the CT scanner, both patient and operator are exposed to radiation for a long time. In contrast, when the interventional treatment is performed by using an ultrasound diagnostic system, the real-time images can be obtained and also it is harmless to the human body. However, an image obtained by using only the ultrasound diagnostic system does not include all lesions and generally shows a portion of each lesion at a low signal-to-noise ratio without its surrounding background. Accordingly, it is difficult to efficiently find a path toward the lesion in the image obtained by using only the ultrasound diagnostic system.

### BRIEF DESCRIPTION OF THE DRAWINGS

Arrangements and embodiments may be described in detail with reference to the following drawings in which like reference numerals refer to like elements and wherein:

FIG. 1 is a block diagram showing an ultrasound system constructed according to one embodiment of the present invention;

FIG. 2 is a perspective view of an exemplary reconstruction image;

FIGS. 3A and 3B are perspective views showing a surrounding area of the lesion seen from different viewpoints;

FIG. 4 shows an explanatory diagram showing a process of setting a medical needle insertion path; and

FIG. 5 illustrates a display example configured according to the embodiment of the present invention.

### DETAILED DESCRIPTION

A detailed description may be provided with reference to accompanying drawings. One of ordinary skill in the art may realize that the following description is illustrative only and is not in any way limiting. Other embodiments of the present invention may readily suggest themselves to such skilled persons having the benefit of this disclosure.

Hereinafter, one embodiment of the present invention will be described with reference to the accompanying drawings.

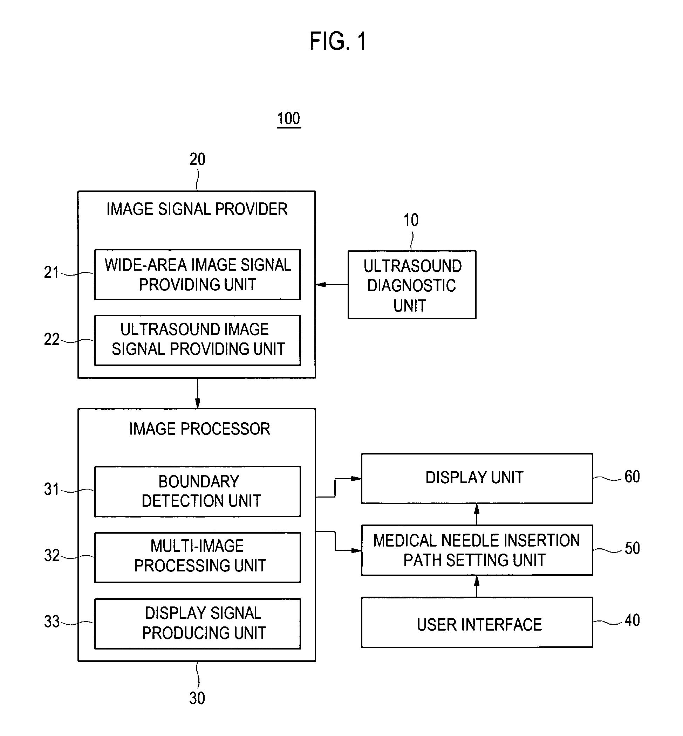

FIG. 1 is a block diagram showing an ultrasound system constructed according to one embodiment of the present invention. As shown in FIG. 1, an ultrasound system 100 includes an ultrasound diagnostic unit 10, an image signal provider 20, an image processor 30, a user interface 40, a medical needle insertion path setting unit (hereinafter, simply referred to as a "path setting unit") 50 and a display unit 60.

The ultrasound diagnostic unit 10 transmits ultrasound signals to a target object. Then, the ultrasound diagnostic unit 10 produces ultrasound image signals based on receive signals reflected from the target object.

The image signal provider 20 includes a wide-area image signal providing unit 21 and an ultrasound image signal providing unit 22. The wide-area image signal providing unit 21 is connected to an imaging system providing an image showing a relatively wide area (hereinafter, referred to as a "wide-area imaging system"), for example, a computerized tomography (CT) scanner or a magnetic resonance imaging (MRI) system. The wide-area image signal providing unit 21 provides wide-area image signals such as CT image signals and MRI image signals to the image processor 30. The wide-area image providing unit 21 may have a memory for storing the wide-area image signals corresponding to a number of frames provided from the wide-area imaging system. Also, the wide-area image providing unit 21 may be data transmission lines.

The ultrasound image signal providing unit 22 provides ultrasound image signals produced by the ultrasound diagnostic unit 10 to the image processor 30. The ultrasound image signal providing unit 22 provides ultrasound image signals acquired previously for planning interventional treatment and ultrasound image signals obtained during the treatment. Specifically, in the treatment planning, the unit 22 provides pre-acquired ultrasound image signals for setting a medical needle insertion path. During the treatment, the unit 22 receives ultrasound image signals of the lesion and the medical needle from the ultrasound diagnostic unit 10 while the medical needle is inserted toward the lesion. Then, the unit 22 provides the received ultrasound image signals to the image processor 30 such that an operator (user) can refer to an ultrasound image of the lesion and the medical needle in the treatment. Similarly to the wide-area image signal providing unit 21, the ultrasound image signal providing unit 22 may have a memory for storing ultrasound image signals corresponding to a number of frames provided from the ultrasound diagnostic unit 10. Also, the ultrasound image signal providing unit 22 may be data transmission lines.

The image processor 30 includes a boundary detection unit 31, a multi-image processing unit 32 and a display signal producing unit 33. The boundary detection unit 31 detects boundaries between objects included in images formed based on the wide-area image signals and the ultrasound image signals provided by the image signal provider 20. The boundaries can be detected by using various well-known image processing methods based on pre-inputted information about organ shapes, intensity of the bone region and the like. The boundary detection unit 31 may be omitted depending on the system.

The multi-image processing unit 32 forms a reconstruction image based on the wide-area image signals (CT image signals or MRI image signals) provided by the wide-area image signal providing unit 21 and the ultrasound image signals provided by the ultrasound image signal providing unit 22. That is, the reconstruction image is a fusion image of the wide-area image and the ultrasound image. Accordingly, since the reconstruction image shows a relatively wide area, a medical needle insertion path for interventional treatment can be accurately and readily set by observing a surrounding area of the lesion. Further, since the reconstruction image shows a real-time image, the operator can watch the process of inserting the medical needle toward the lesion during the treatment.

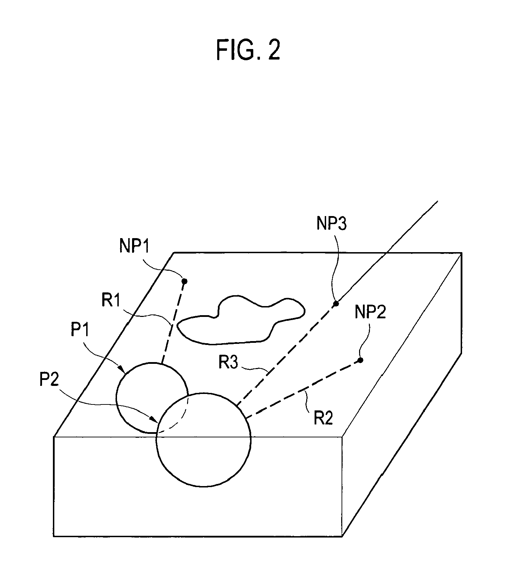

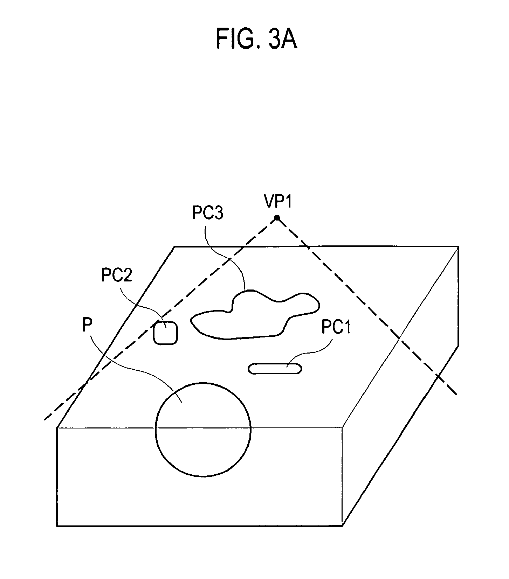

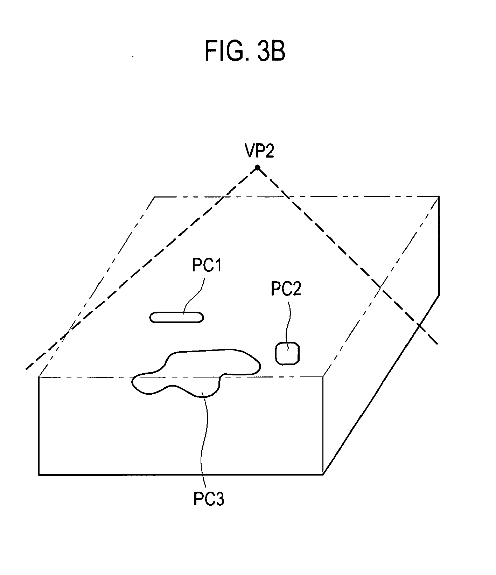

The reconstruction image may be a three-dimensional (3D) image as shown in FIG. 2. Further, the reconstruction image may show a surrounding area of the lesion seen from different viewpoints VP1 and VP2 as shown in the FIGS. 3A and 3B. FIG. 3A illustrates a perspective view of a 3D reconstruction image including a lesion P and substances PC1, PC2 and PC3, seen from the skin to the lesion. FIG. 3B illustrates a perspective view of a 3D reconstruction image including the substances PC1, PC2 and PC3, seen from the lesion P to the skin. By providing various views of the reconstruction image such as those shown in FIGS. 3A and 3B, the medical needle insertion path can be set by using various information.

On the reconstruction image, the user can select at least one lesion, medical needle insertion point (hereinafter, simply referred to as "insertion point"), an arbitrary point for changing the medical needle insertion path (hereinafter, referred to as "designated point") and the like. The information regarding the selected lesion, designated point and insertion point is displayed in the display unit 60 as text data.

The display signal producing unit 33 produces display signals for controlling the number, positions and sizes of display windows and then provides them to the display unit 60. Accordingly, the reconstruction image, wide-area image and, ultrasound image and text data can be displayed at the same time in the display unit 60. The text data include positions and the number of the lesions, insertion points and designated points and the medical needle insertion paths.

The user interface 40 may allow the user to select the lesion, medical needle insertion point, arbitrary point and the like on the reconstruction image. The user interface 40 may be configured as a mouse, track ball or the like. As shown in FIG. 2, the user selects lesions P1 and P2 and insertion points NP1, NP2 and NP3 through the user interface 40. For example, the lesions P1 and P2 and insertion points NP1, NP2 and NP3 may be represented as spheres and points on the reconstruction image, respectively.

The path setting unit 50 receives information regarding the positions and the number of lesions, designated points and insertion points which are selected by the user through the user interface 40. Then, the path setting unit 50 computes coordinates of the lesions, designated points and insertion points and sizes of the lesions. The path setting unit 50 offers a medical needle insertion path based on the computed coordinates. For example, as shown in FIG. 2, the path setting unit 50 sets paths R1, R2 and R3 based on a minimum distance between lesion P1 and an insertion point NP1, a minimum distance between a lesion P2 and an insertion point NP2 and a minimum distance between the lesion P2 and an insertion point NP3. The set paths R1, R2 and R3 are offered to the user by displaying it on the reconstruction image

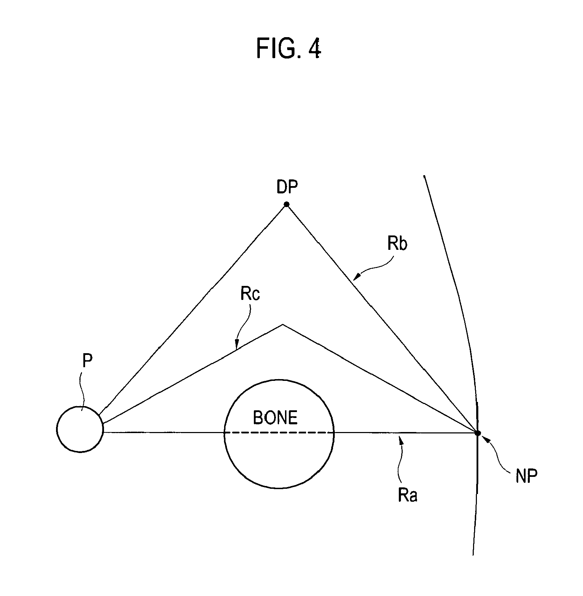

Meanwhile, as shown in FIG. 4, when a path Ra is set based on a minimum distance between a lesion P and an insertion point NP, the medical needle should be inserted through the bone. In this case, for example, the user may select a designated point DP to change the medical needle insertion path. Accordingly, the path setting unit 50 resets a path Rb based on positions of the designated point DP, the lesion P and the insertion point NP.

Further, in the case shown in FIG. 4, the path setting unit 50 may offer an arbitrary path based on the positions of the lesion P, the insertion point NP and boundaries detected by the boundary detection unit 31. Image signals of neighboring structures such as bone or tissue passing through the path Ra are produced by using well-known techniques, for example, MPR (Multi-Planar Reconstruction), 3D shaded/unshaded volume rendering, MIP (Maximum Intensity Projection), MinIP (Minimum Intensity Projection), SSD (Shaded Surface Display), and Virtual endoscopy. Then, based on the image signals of neighboring structures, the path setting unit 50 may offer a path Rc as shown in FIG. 4. The user can select a final path among the offered paths or directly set the medical needle insertion path on the reconstruction image through the user interface 40.

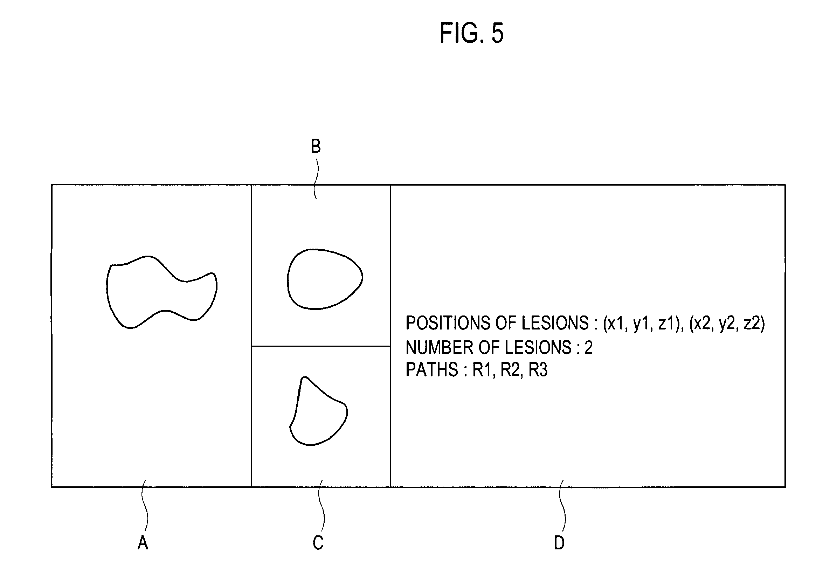

The display unit 60 displays the reconstruction image, the wide-area image and the ultrasound image which are formed in the image processor 30. The display unit 60 also displays the text data. FIG. 5 shows an example of displaying the reconstruction image, the wide-area image and the ultrasound image in the display windows A, B and C, respectively, and also displaying the text data including positions and the number of lesions and paths in the text window D. The number of display windows and images displayed in the respective windows can change depending on setting conditions of the system.

On the other hand, the display unit 60 may be provided with image signals of a neighboring structure from the image processor 30. Then, it may display an image of the neighboring structure in one of the display windows A, B and C or an additional display window.

Further, the display unit 60 may display the final path as a guide line on the real-time ultrasound image showing a process of inserting the medical needle toward the lesion during the treatment. Thus, the user can check whether the medical needle is inserted correctly along the final path.

As describe above, the ultrasound system in accordance with the present invention provides various views of the reconstruction image formed based on the CT or MRI image showing a relatively wide area. Thus, the operator can set the medical needle insertion path before the treatment while accurately and readily observing the surrounding area of the lesion. Further, the ultrasound system can provide the user with the real-time image showing a medical needle insertion process. Thus, the operator can check whether the medical needle is inserted along the preset path.

An embodiment may be achieved in a whole or in parts by an ultrasound system, which includes: an ultrasound diagnostic unit for transmitting ultrasound signals to a target object and producing ultrasound image signals based on receive signals reflected from the target object; a wide-area image signal providing unit for providing wide-area image signals for a relatively wide area; an image processor for forming a reconstruction image by combining an ultrasound image and a wide-area image based on the ultrasound image signals and the wide-area image signals; a user interface for selecting at least one lesion in the target object and at least one medical needle insertion point by a user; a path setting unit for setting at least one medical needle insertion path based on the lesion and the medical needle insertion point; and a display unit for displaying the reconstruction image showing positions of the lesion and the medical needle insertion point and the medical needle insertion path, the display unit further being configured to display the ultrasound image in real time

Any reference in this specification to "one embodiment," "an embodiment," "example embodiment," etc., means that a particular feature, structure, or characteristic described in connection with the embodiment is included in at least one embodiment of the invention. The appearances of such phrases in various places in the specification are not necessarily all referring to the same embodiment. Further, when a particular feature, structure, or characteristic is described in connection with any embodiment, it is submitted that it is within the purview of one skilled in the art to effect such feature, structure, or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound diagnostic unit for transmitting ultrasound signals to a target object and producing ultrasound image signals based on receive signals reflected from the target object;
a wide-area image signal providing unit for providing wide-area image signals for a relatively wide area;
an image processor for forming a reconstruction image by combining an ultrasound image and a wide-area image based on the ultrasound image signals and the wide-area image signals;
a user interface for selecting at least one lesion in the target object and at least one medical needle insertion point by a user;
a path setting unit for setting at least one medical needle insertion path based on the lesion and the medical needle insertion point; and
a display unit for displaying the reconstruction image showing positions of the lesion and the medical needle insertion point and the medical needle insertion path, the display unit further being configured to display the ultrasound image in real time.

2. The ultrasound system of Claim 1, wherein the wide-area image signals are produced by a computerized tomography (CT) scanner.

3. The ultrasound system of Claim 1, wherein the wide-area image signals are produced by a magnetic resonance imaging (MRI) system.

4. The ultrasound system of Claim 1, wherein the reconstruction image is obtained from various viewpoints.

5. The ultrasound system of Claim 4, wherein the reconstruction image includes the lesion and its neighboring structures, seen from a surface of the target object to the lesion.

6. The ultrasound system of Claim 4, wherein the reconstruction image includes neighboring structures of the lesion, seen from the lesion to a surface of the target object.

7. The ultrasound system of Claim 1, wherein the user further selects at least one designated point through the user interface and the path setting unit resets the medical needle insertion path based on the lesion, the medical needle insertion point and the designated point.

8. The ultrasound system of Claim 1, further comprising a boundary detecting unit for detecting boundaries between the objects based on the wide-area image signals and the ultrasound image signals.

9. The ultrasound system of Claim 8, wherein the path setting unit resets the medical needle insertion path based on the lesion, the medical needle insertion point and the detected boundaries.

10. The ultrasound system of Claim 1, wherein the display unit displays the medical needle insertion path on the ultrasound image displayed in real time.
